# EUROPEAN PATENT SPECIFICATION

(11) **EP 1 516 601 B1**
(45) Date of publication and mention of the grant of the patent: **12.11.2008**
(21) Application number: 04255121.8
(22) Date of filing: 25.08.2004
(51) Int. Cl.: A61F 2/06, A61F 2/01

(54) **Self-expanding stent and stent and delivery system with distal protection**
Selbstexpandierbarer Stent, Stent und Einführsystem mit distalem Schutz
Stent auto-expansible, stent et système de mise en place avec protection distale

(30) Priority: 29.08.2003 US 651605
(43) Date of publication of application: 23.03.2005
(73) Proprietor: Cordis Neurovascular, Inc., Miami Lakes, Florida 33014 (US)
(72) Inventor: Jones, Donald K., Lauderhill Florida 33351 (US); Mitelberg, Vladimir, Aventura Florida 33180 (US)
(74) Representative: Tunstall, Christopher Stephen

(56) References cited:
- EP-A- 1 025 813
- EP-A- 1 362 564
- WO-A-03/039405
- US-A1- 2002 161 389
- US-A1- 2003 065 356
- US-B1- 6 174 327

## Description

### Background of the Invention

### Field of the Invention

This invention relates to intravascular stents, stent delivery systems, and methods of treating a stenosis within a blood vessel. More specifically, this invention relates to stent delivery systems having integral distal protection devices for care in percutaneous transluminal angioplasty of occluded blood vessels within the brain of a patient.

### Description of the Prior Art

On a worldwide basis, nearly one million balloon angioplasties are performed annually to treat vascular diseases such as blood vessels that are clogged or narrowed by a lesion or stenosis. The objective of this procedure is to increase the inner diameter of the partially occluded blood vessel lumen. In an effort to prevent restenosis without requiring surgery, short flexible cylinders or scaffolds, referred to as stents, are often placed into the blood vessel at the site of the stenosis.

Stents are typically made of metal or polymers and are widely used for reinforcing diseased blood vessels. Some stents are expanded to their proper size using a balloon catheter. Such stents are referred to as "balloon expandable" stents. Other stents, referred to as "self-expanding" stents, are designed to elastically resist compression in a self-expanding manner. Balloon expandable stents and self-expanding stents are compressed into a small diameter cylindrical form and deployed within a blood vessel using a catheter-based delivery system, such as a balloon catheter.

Several balloon catheters have been disclosed in prior patents. One such balloon catheter is disclosed in U.S. Patent No. 5,843,090, entitled "Stent Delivery Device," wherein a balloon catheter, having inner and outer catheters, with the outer catheter having a second lumen for inflation of a balloon, is used as a stent delivery device. U.S. Patent No. 5,639,274, entitled "Integrated Catheter System for Balloon Angioplasty and Stent Delivery," discloses an integrated catheter system including a stent catheter and a balloon angioplasty catheter, where the stent catheter contains a stent and is displaced over the balloon catheter. However, current balloon catheters are typically too large and inflexible to traverse the tortuous blood vessels within the brain.

Recently, filters mounted on the distal end of guidewires have been proposed for intravascular blood filtration during balloon angioplasty and the delivery of vascular stents. One such filter is disclosed in U.S. Patent No. 6,168,579, entitled "Filter Flush System and Methods of Use." This patent discloses a filter flush system for temporary placement of a filter in a blood vessel. The filter system includes a guidewire having an expandable filter which may be collapsed to pass through the lumen of a guiding catheter and may then be expanded upstream of a stenosis prior to angioplasty or to the placement of a stent. U.S. Patent Application Publication No. 2002/0115942, entitled "Low Profile Emboli Capture Device," discloses an emboli capture device comprised of a filter and a self-expanding stent. The self-expanding stent is attached to the filter in order to open the filter when the emboli capture device is placed within an artery. U.S. Patent No. 6,027,520, entitled "Percutaneous Catheter and Guidewire Having Filter and Medical Device Deployment Capabilities," discloses a percutaneous catheter including a filter and a stent deployed on an inflatable balloon or stent catheter.

### Summary of the Invention

In accordance with one aspect of the present invention, there is provided a self-expanding stent and stent delivery system including a deployment catheter, an elongated core member slidably disposed within the lumen of the deployment catheter, a self-expanding stent mounted on said elongated core member, and an expandable capture basket attached to the distal end of the elongated core member. The expandable capture basket is comprised of a self-expanding metallic frame and a mesh body. The expandable capture basket is spaced apart from the self-expanding stent and serves the purpose of trapping embolic debris which may be dislodged from a diseased blood vessel during placement of the self-expanding stent within the diseased blood vessel. The distance between the proximal end of the capture basket and the distal end of the self-expanding stent is in a range of about one millimeter to two centimeters, but preferably in a range of five millimeters to fifteen millimeters. Additionally, the elongated core member preferably includes a stop member extending radially outward from the elongated core member to engage the self-expanding stent to the elongated core member.

In accordance with another aspect of the present invention, there is provided a self-expanding stent and stent delivery system including a balloon catheter taking the form of an elongated catheter having a delivery lumen and an expandable balloon mounted on the distal section of the elongated catheter. The stent delivery system further includes an elongated core member having a stop member extending radially outward from the elongated core member. The elongated core member is slidably disposed within the delivery lumen of the elongated catheter. A self-expanding stent is mounted on the elongated core member, engaging the stop member so that the self-expanding stent may be moved through the delivery lumen when the elongated core member is moved through the delivery lumen of the elongated catheter.

Additionally, an expandable capture basket is attached to the distal end of the elongated core member to serve the purpose of trapping embolic debris which may be dislodged from a diseased blood vessel during angioplasty of the blood vessel. The expandable capture basket is comprised of a self-expanding metallic frame and a mesh body. Furthermore, the expandable capture basket is spaced apart from the self-expanding stent such that the proximal end of the capture basket is at a distance from the distal end of the stent. The distance between the proximal end of the expandable capture basket and the distal end of the self-expanding stent is in a range of about one millimeter to two centimeters, but preferably in a range of about five millimeters to fifteen millimeters.

In accordance with another aspect of the present invention, the elongated core member takes the form of a wire. In this embodiment, the stop member takes the form of a cylindrical coil disposed about the elongated core member.

In accordance with still another aspect of the present invention, there is provided a self expanding stent and stent delivery system comprising a balloon catheter taking the form of an elongated catheter having a delivery lumen and an expandable balloon mounted on the distal section of the elongated catheter. An elongated core member is slidably disposed within the delivery lumen of the elongated catheter. A self-expanding stent is mounted on the elongated core member. The elongated core member further includes a stop member extending radially outward from the elongated core member. The self-expanding stent engages with the stop member to thereby allow the self-expanding stent to be moved proximally and distally through the delivery lumen of the elongated catheter when the elongated core member is moved proximally and distally through the delivery lumen of the elongated catheter. Additionally, a self-expanding capture basket is attached to the distal end of the elongated core member and spaced apart from the self-expanding stent.

In accordance with yet another aspect of the present invention, there is provided a self-expanding stent and stent delivery system including a deployment catheter, an elongated core member slidably disposed within the lumen of the deployment catheter, and a self-expanding stent mounted on the elongated core member. The elongated core member preferably takes the form of a wire including a stop member which aids in engaging the self-expanding stent to the elongated core member. The stent delivery system further includes a self-expanding capture basket attached to the distal end of the elongated core member. The self-expanding capture basket serves the purpose of trapping embolic debris which may be dislodged from a diseased blood vessel during placement of the self-expanding stent. Additionally, the self-expanding capture basket is comprised of a self-expanding metallic frame and a mesh body.

In accordance with another aspect of the present invention, there is provided a method of treating a stenosis comprising the steps of inserting a balloon catheter into a vessel of a patient, advancing the balloon catheter until the balloon catheter is positioned across a stenosis within the vessel, inserting a self-expanding stent mounted on an elongated core member into the delivery lumen of the balloon catheter and advancing the self-expanding stent and elongated core member distally through the delivery lumen until the self-expanding stent is aligned approximate the stenosis. The method further includes the steps of expanding the balloon and withdrawing the catheter proximally to thereby allow a self-expanding capture basket and the self-expanding stent to expand within the vessel. The self-expanding stent is then disengaged from the elongated core member and the balloon catheter and elongated core member are withdrawn from the vessel of the patient.

In accordance with a further aspect of the present invention, there is provided a method of treating a stenosis comprising the steps of inserting a balloon catheter into a blood vessel of a patient over a guidewire, advancing the guidewire and the balloon catheter until the balloon catheter is positioned across a stenosis within the blood vessel, and removing the guidewire. The method further includes the steps of inserting a self-expanding stent mounted on an elongated core member into the delivery lumen of the catheter, the core member having a capture basket attached to its distal end, and advancing the self-expanding stent and elongated core member distally through the delivery lumen until the self-expanding stent is aligned approximate the stenosis and the capture basket has exited the delivery lumen and expanded within the blood vessel. Then, fluid is injected into the inflation lumen of the balloon catheter to thereby inflate the balloon. The method continues by removing the fluid from within the inflation lumen to thereby deflate the balloon, withdrawing the balloon catheter proximally to thereby allow the self-expanding stent to expand within the blood vessel, disengaging the self-expanding stent from the core member, retracting the elongated core member so that the capture basket collapses within the delivery lumen, and withdrawing the balloon catheter and the core member from the blood vessel of the patient.

In accordance with another aspect of the present invention, there is provided a method of treating a stenosis comprising the steps of inserting a deployment catheter into a vessel of a patient over a guidewire, advancing the guidewire and the deployment catheter until the deployment catheter is positioned across a stenosis within the blood vessel, removing said guidewire, inserting a self-expanding stent mounted on an elongated core member into the lumen of the deployment catheter, and advancing the self-expanding stent and elongated core member distally through the lumen until the self-expanding stent is aligned approximate the stenosis and an expandable capture basket has exited the lumen and expanded within the blood vessel. The method further includes the steps of withdrawing the deployment catheter proximally to thereby allow the self-expanding stent to expand within the blood vessel, disengaging the self-expanding stent from the core member, retracting the elongated core member until the capture basket has collapsed into the lumen of the deployment catheter, and withdrawing the deployment catheter and the elongated core member from the blood vessel of the patient.

### Brief Description of the Drawings

Figure 1 is a partially sectioned view of a balloon catheter containing a self-expanding stent mounted on an elongated core wire, said wire including a capture basket attached to its distal end;
Figure 2 is a sectioned view of the elongated core wire of Figure 1 having a self-expanding stent mounted on the elongated core wire and having a capture basket attached to the distal end of the core wire;
Figure 3 is a sectioned view of the balloon catheter of Figure 1 within a blood vessel;

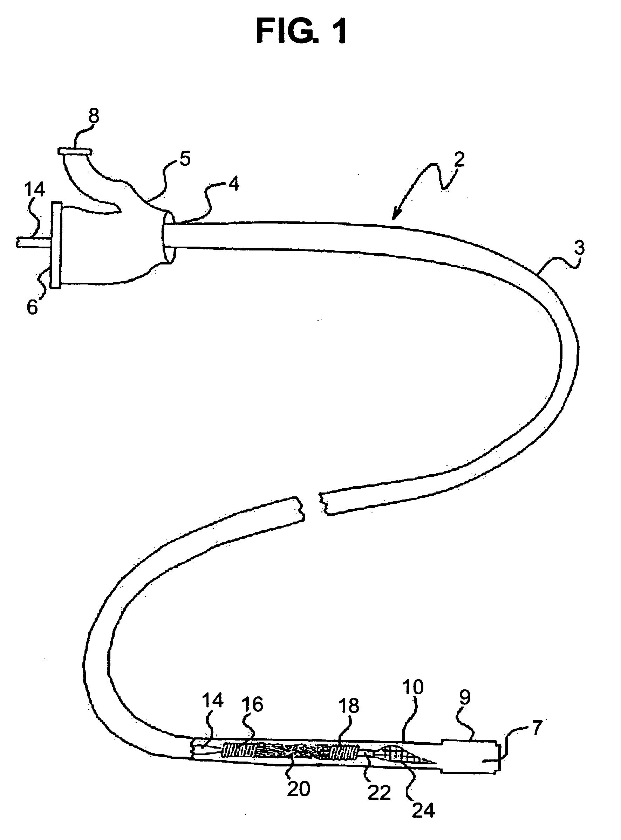

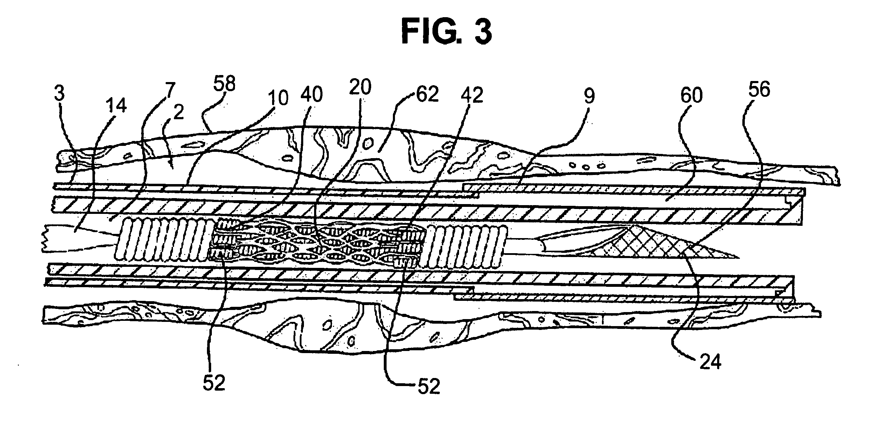

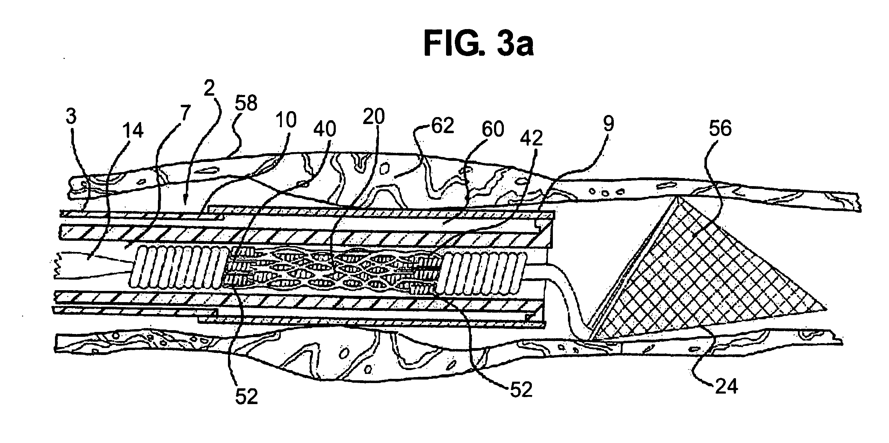

Figure 3 a is a sectioned view of the balloon catheter of Figure 1 within a blood vessel and having the capture basket deployed and expanded within the blood vessel;

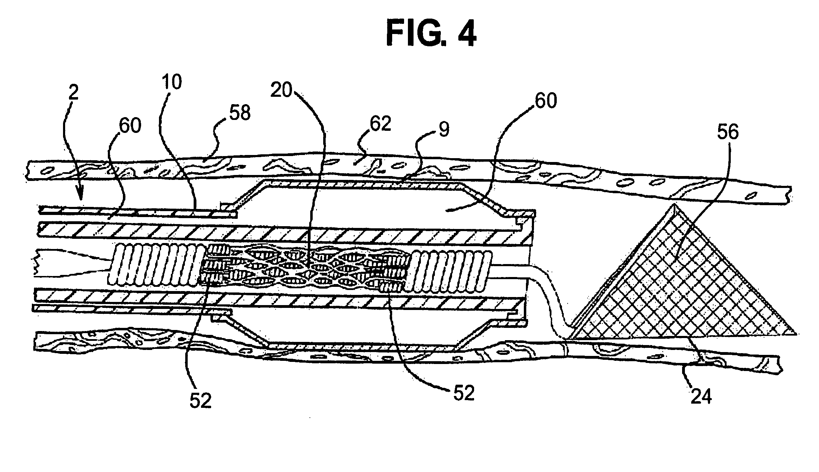

Figure 4 is a sectioned view of the balloon catheter, within the blood vessel, having the balloon fully expanded;

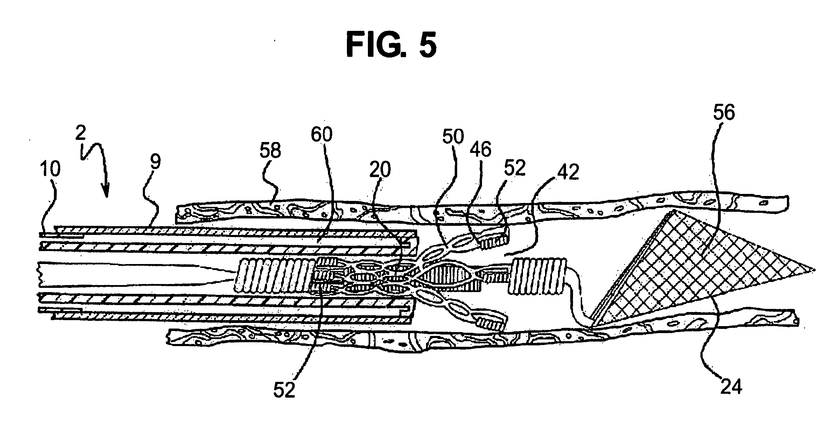

Figure 5 is a sectioned view of the balloon catheter being moved proximally thereby allowing the self-expanding stent to begin expanding within the blood vessel;

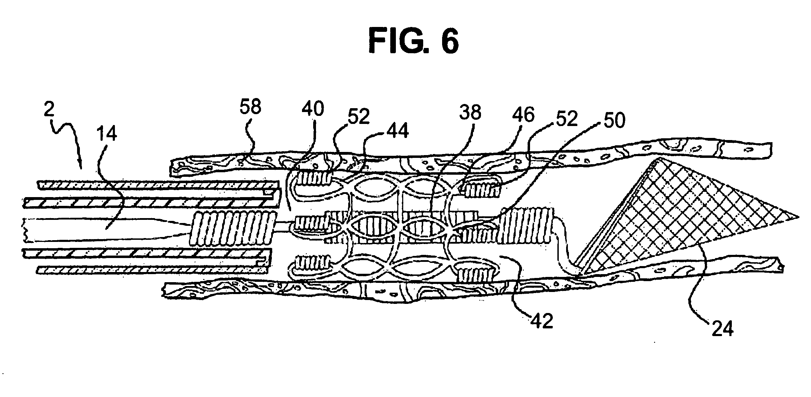

Figure 6 is a sectioned view of the self-expanding stent fully expanded within the blood vessel while the elongated core wire remains extended through the stent;

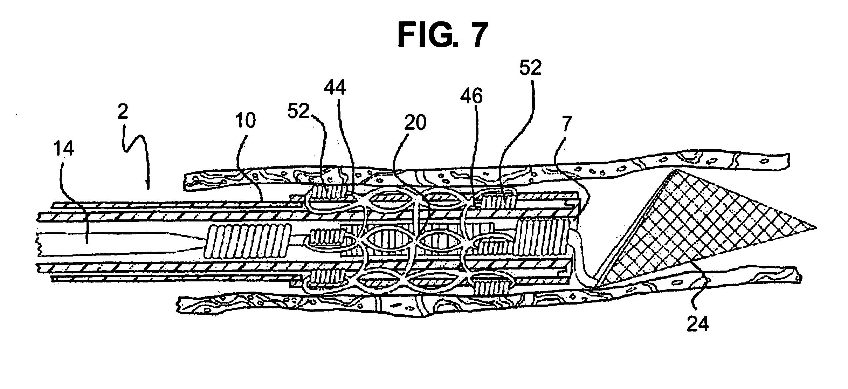

Figure 7 is a sectioned view of the balloon catheter advanced distally over the core wire and through the self-expanding stent;

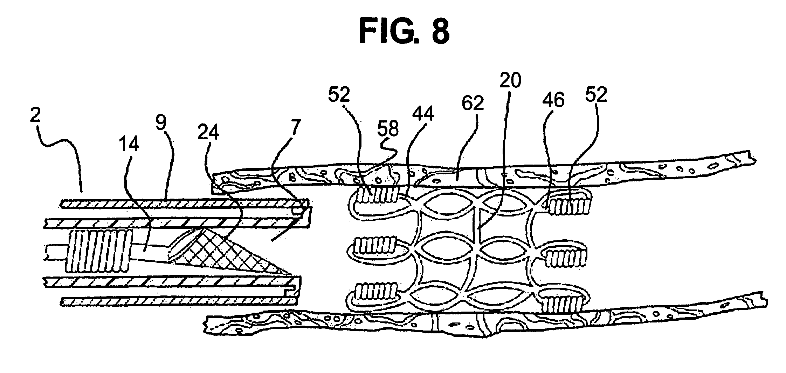

Figure 8 is a sectioned view of the balloon catheter and elongated core wire withdrawn proximally through the self-expanding stent; and,

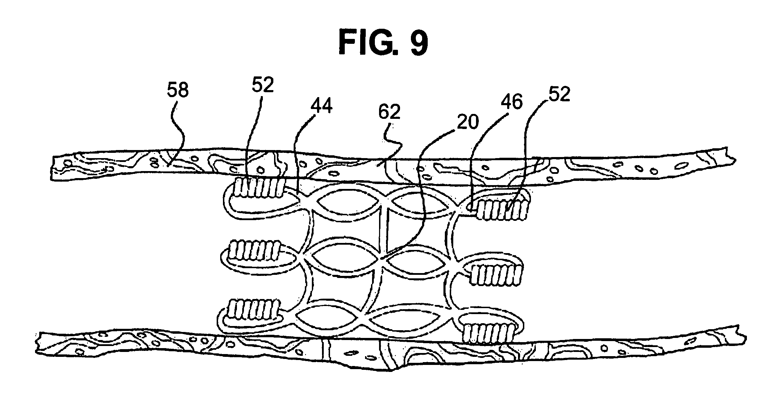

Figure 9 is a view of the self-expanding stent within the blood vessel with the balloon catheter and elongated core wire removed from within the blood vessel.

### Description of the Preferred Embodiment

Figure 1 illustrates a balloon catheter 2 comprising an elongated outer catheter 3. Attached to the proximal end 4 of the outer catheter 3 is a coupling member 5. The coupling member 5 includes a delivery port 6 which communicates with a delivery lumen 7, which extends throughout the length of the balloon catheter 2. The coupling member 5 also includes an activation port 8 used to activate and expand an expandable balloon 9 disposed about the distal portion 10 of the outer catheter 3. The balloon catheter 2 should be rigid enough to be pushed distally through a blood vessel, yet flexible enough to traverse the narrow and tortuous blood vessels within the brain.

Slidably disposed within the delivery lumen 7 is an elongated core member 14, preferably taking the form of an elongated core wire. Disposed about the elongated core wire 14 are a proximal cylindrical member 16 and a distal cylindrical member 18. A self-expanding stent 20 is mounted on the elongated core wire 14. The proximal and distal cylindrical members 16,18 serve as stop members extending radially outward from the core wire 14 to engage the stent 20 with the elongated core wire such that the stent can be moved proximally and distally through the delivery lumen 7. Attached to the distal end 22 of the elongated core wire 14 is an expandable capture basket 24.

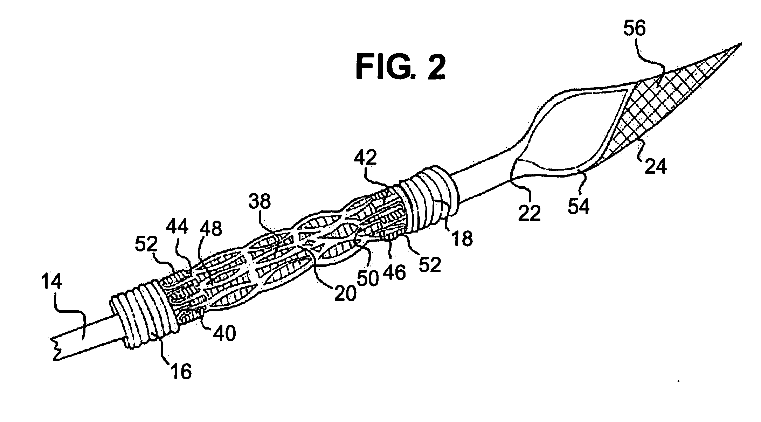

Figure 2 illustrates the self-expanding stent 20 mounted on the elongated core wire 14. Disposed about the elongated core wire 14 is a proximal cylindrical member 16. Preferably, the proximal cylindrical member 16 is a helically wound flexible coil made of metal, but may alternatively be formed of a polymer material. An intermediate cylindrical member 38 (shown within the stent) is also disposed about the core wire 14 and is generally positioned distally from the proximal cylindrical member 16. The intermediate cylindrical member 38 is spaced apart from the proximal cylindrical member 16 such that the space between the proximal and intermediate cylindrical members 16, 38 forms a first gap 40.

A distal cylindrical member 18 is disposed about the elongated core wire 14 and is generally positioned distally from the intermediate cylindrical member 38. The distal cylindrical member 18 is spaced apart from the intermediate cylindrical member 38 such that the space between the intermediate and distal cylindrical members 38, 18 forms a second gap 42. Preferably, the distal cylindrical member 18 is a helically wound flexible coil made from metal, but may alternatively be formed of a polymer material.

Mounted on the intermediate cylindrical member 38, the self-expanding stent 20 may take on many different patterns or configurations. Examples of such stents are disclosed in two U.S. Patent Applications, both entitled "Intravascular Stent Device," filed June 5, 2002, and having U.S. Serial Nos. 10/163,116 and 10/163,248 and assigned to the same assignee as the present patent application. Preferably, the stent 20 is coated with an agent, such as heparin or rapamycin, to prevent stenosis or restenosis of the vessel. Examples of such coatings are disclosed in U.S. Patent Nos. 5,288,711; 5,516,781; 5,563,146 and 5,646,160.

The self-expanding stent 20 is preferably laser cut from a tubular piece of nitinol to form a skeletal tubular member. The skeletal tubular member has a small diameter and a thin wall comprised of a plurality of cells which are formed by a plurality of interconnected strut members. Then, the nitinol is treated so as to exhibit superelastic properties at body temperature. Additionally, the stent 20 includes proximal and distal strut members 44, 46 coupled to the proximal and distal sections 48, 50 of the stent. Preferably, the proximal and distal strut members 44, 46 are cut to form threads on the strut members during the laser-cutting of the stent 20 from the tubular piece of nitinol. Radiopaque coils are then wound onto the threads of the proximal and distal strut members 44, 46 to form anchor members 52. Preferably, the stent 20 includes eight anchor members 52. When the self-expanding stent 20 is mounted on the elongated core wire 14, the anchor members 52 align with and are disposed within the first and second gaps 40, 42 thus engaging the stent with the elongated core wire. In this configuration, the stent 20 can be moved distally and proximally through the delivery lumen 7 of the balloon catheter 2. The self-expanding stent 20 is described in more detail in U.S. Patent Application, entitled "Expandable Stent with Radiopaque Markers and Stent Delivery System," filed on June 27, 2003 (Attorney Docket No. CRD-5001-US-CIP) and assigned to the same assignee as the present patent application.

Attached to the distal end 22 of the elongated core wire 14 is the capture basket 24. The capture basket 24 is spaced apart from the self-expanding stent 20. The distance between the proximal end of the capture basket 24 and the distal end of the self-expanding stent 20 is in a range of about one millimeter to two centimeters, but preferably in a range of about five millimeters to fifteen millimeters. The capture basket 24 is preferably comprised of a self-expanding metallic frame 54 and a mesh body 56. The metallic frame 54 is designed to collapse within the delivery lumen 7 of the balloon catheter 2, yet be capable of expanding and covering a blood vessel upon deployment. The mesh body 56 is intended to capture any embolic debris released during angioplasty of the blood vessel and the deployment of the self-expanding stent 20 within the blood vessel.

Figure 3 shows the balloon catheter 2 inserted within a blood vessel 58 of the brain of a patient. The balloon catheter 2 includes an expandable balloon 9 disposed about the distal portion 10 of the elongated outer catheter 3. In the preferred embodiment of the present invention, an inflation lumen 60 extends from the activation port 8 and communicates with the balloon 9. To perform an angioplasty of the blood vessel 58, a fluid is injected into the inflation lumen 60, through the activation port 8, to thus expand the balloon 9. The balloon catheter 2 is described in more detail in U.S. Patent No. 6,585,687, entitled "Inflatable Balloon Catheter Body Construction," assigned to the same assignee as the present patent application.

Typically, the balloon catheter 2 is advanced distally through the blood vessel 58 over a guidewire until it is aligned with a stenosis 60. Then, the guidewire is removed and the elongated core wire 14 is inserted into the delivery lumen 7 of the balloon catheter 2. The self-expanding stent 20 is mounted on the elongated core wire 14 such that the anchor members 52 align with and are disposed within the first gap 40, between the proximal and intermediate cylindrical members 16, 38, and the second gap 42, between the intermediate and distal cylindrical members 38, 18. In this configuration, the stent 20 is engaged to the core wire 14 so that the stent may be moved proximally and distally through the delivery lumen 7 of the balloon catheter 2.

As shown in Figure 3a, the elongated core wire 14 is advanced distally through the delivery lumen 7 of the balloon catheter 2 until the capture basket 24 has exited the delivery lumen and fully expanded within the blood vessel 58 distal of the stenosis 62. With the capture basket 24 fully deployed within the blood vessel 58, any embolic debris released from the stenosis 62 will be captured within the mesh body 56 of the capture basket, and thus removed from the blood vessel after the completion of the procedure.

Figure 4 illustrates the balloon catheter 2 having the expandable balloon 9 fully expanded within the blood vessel 58. Preferably, the balloon 9 is expanded by injecting fluid into the inflation lumen 60 of the balloon catheter. The expanded balloon 9 compresses the stenosis 62 and thus increases the luminal diameter of the blood vessel 58. During the compression of the stenosis 62, embolic debris may dislodge from the stenosis and flow down the blood stream. In this case, the capture basket 24 will filter the blood and collect any embolic debris in the blood stream.

In Figure 5, the balloon 9 is contracted and the balloon catheter 2 is moved proximally, releasing anchor members 52 on the distal strut members 46 from the second gap 42 and allowing the distal section 50 of the self-expanding stent 20 to begin expanding. During expansion, the distal section 50 of the stent 20 comes in contact with the wall of the blood vessel 58.

As illustrated in Figure 6, the balloon catheter 2 is again moved proximally, releasing the anchor members 52 on the proximal strut members 44 from the first gap 40 and allowing the proximal section 48 of the self-expanding stent 20 to expand. Once the stent 20 is fully deployed within the blood vessel 58, the core wire 14 remains extended through the stent 20 and thus serves as a guidewire, providing a physician with easier access to locations within the blood vessel distal of the stent.

If, during the deployment process, it is determined that the stent 20 should be relocated or realigned, the balloon catheter 2 may be used to resheath the stent 20. With the stent 20 mounted on the core wire 14 as described above, if the balloon catheter 2 is not withdrawn beyond the anchor members 52 on the proximal strut members 44, the stent will remain interlocked on the core wire 14. In this configuration, the stent 20 may be resheathed. To resheath the stent 20, the balloon catheter 2 is moved distally forcing the stent back onto the intermediate cylindrical member 38, compressing the distal section 50 of the stent, and forcing the anchor members 52 on the distal strut members 46 to become interlocked within the second gap 42. The stent 20 and balloon catheter 2 may then be withdrawn or repositioned to a different location within the blood vessel 58.

Figure 7 illustrates the balloon catheter 2 advanced distally over the elongated core wire 14 and through the self-expanding stent 20. The balloon catheter 2 is advanced distally until the capture basket 24 has collapsed within the delivery lumen 7 of the balloon catheter.

As shown in Figure 8, when the capture basket 24 collapses within the delivery lumen 7 of the balloon catheter 2, the balloon catheter and elongated core wire 14 may be removed from within the blood vessel 58. In this fashion, embolic debris captured within the capture basket 24 can be retained and removed from within the blood vessel. The capture basket thus prevents dislodgements from the preceding procedure to travel down the blood stream and create further complications such as ischemic strokes.

Figure 9 shows the self-expanding stent 20 fully expanded within the blood vessel 58 with the balloon catheter 2 removed from within the blood vessel. The stent 20 compresses the stenosis 62 and thus aids in preventing restenosis.

A novel system has been disclosed in which a self-expanding stent is mounted on an elongated core member and is slidably disposed within a balloon catheter. Although a preferred embodiment of the present invention has been described, it is to be understood that various modifications may be made by those skilled in the art without departing from the scope of the claims which follow.

## Claims

1. A self-expanding stent and stent delivery system comprising:
a balloon catheter (2) comprised of an elongated catheter (3) having a delivery lumen (7), proximal and distal sections (4, 10), and an expandable balloon (9) mounted on the distal section (10) of said elongated catheter (3);
an elongated core member (14) having proximal and distal (22) ends, said elongated core member (14) slidably disposed within the delivery lumen (7) of said elongated catheter (3);
a self-expanding stent (20) mounted on said elongated core member (14); and,
an expandable capture basket (24) attached to the distal end (22) of said elongated core member (14), said expandable capture basket (24) serving the purpose of trapping embolic debris which may be dislodged from a diseased blood vessel during angioplasty of the blood vessel.

2. A self-expanding stent and stent delivery system as defined in Claim 1, wherein said elongated core member (14) includes a stop member (16, 18) extending radially outward from said elongated core member (14), and said self-expanding stent (20) engages with said stop member (16, 18) to thereby allow said self-expanding stent (20) to be moved proximally and distally through said delivery lumen (7) of said elongated catheter (3) when said elongated core member (14) is moved proximally and distally through said delivery lumen (7) of said elongated catheter (3).

3. A self-expanding stent and stent delivery system as defined in either Claim 1 or Claim 2, wherein said expandable capture basket (24) is a self-expanding capture basket (24).

4. A self-expanding stent and stent delivery system as defined in any of Claims 1 to 3, wherein said expandable capture basket (24) is comprised of a self-expanding metallic frame (54) and a mesh body (56).

5. A self-expanding stent and stent delivery system as defined in Claim 4, wherein said expandable capture basket (24) includes proximal and distal ends, said self-expanding stent (20) includes proximal and distal ends, and said self-expanding stent (20) is mounted on said elongated core member (14) proximal of said expandable capture basket (24) such that the proximal end of said capture basket (24) is spaced apart from the distal end of said stent (20).

6. A self-expanding stent and stent delivery system as defined in Claim 5, wherein the distance between the proximal end of said expandable capture basket (24) and the distal end of said self-expanding stent (20) is in a range of about one millimeter to two centimeters.

7. A self-expanding stent and stent delivery system as defined in Claim 5, wherein the distance between the proximal end of said expandable capture basket (24) and the distal end of said self-expanding stent (20) is in a range of about five millimeters to fifteen millimeters.

8. A self-expanding stent and stent delivery system as defined in Claim 5, wherein said elongated core member (14) takes the form of a wire.

9. A self-expanding stent and stent delivery system as defined in Claim 8, wherein said stop member (16, 18) takes the form of a cylindrical coil disposed about said elongated core member (14).

## Patentansprüche

1. Selbstexpandierender Stent und Stentzuführungssystem, die Folgendes umfassen:
einen Ballonkatheter (2), der einen länglichen Katheter (3) mit einem Zuführlumen (7), einen proximalen und einen distalen Abschnitt (4, 10) und einen expansionsfähigen Ballon (9), der an dem distalen Abschnitt (10) des länglichen Katheters (3) montiert ist, umfasst;
ein längliches Kemelement (14) mit einem proximalen und einem distalen (22) Ende, wobei das längliche Kemelement (14) gleitfähig in dem Zuführungslumen (7) des länglichen Katheters (3) angeordnet ist;
einen selbstexpandierenden Stent (20), der an dem länglichen Kemelement (14) montiert ist; und
einen expansionsfähigen Einfangkorb (24), der an dem distalen Ende (22) des länglichen Kernelements (14) angebracht ist, wobei der expansionsfähige Einfangkorb (24) dem Zweck dient, Embolietrümmer zu fangen, welche sich von einem erkrankten Blutgefäß während einer Angioplastie des Blutgefässes gelöst haben können.

2. Selbstexpandierender Stent und Stentzuführungssystem nach Anspruch 1, wobei das längliche Kemelement (14) ein Anschlagelement (16, 18) umfasst, das sich von dem länglichen Kemelement (14) radial nach außen erstreckt, und wobei der selbstexpandierende Stent (20) an dem Anschlagelement (16, 18) eingreift, um dabei zu ermöglichen, dass der selbstexpandierende Stent (20) proximal und distal durch das Zuführungslumen (7) des länglichen Katheters (3) bewegt wird, wenn das längliche Kemelement (14) proximal und distal durch das Zuführungslumen (7) des länglichen Katheters (3) bewegt wird.

3. Selbstexpandierender Stent und Stentzuführungssystem nach Anspruch 1 oder 2, wobei der expansionsfähige Einfangkorb (24) ein selbstexpandierender Einfangkorb (24) ist.

4. Selbstexpandierender Stent und Stentzuführungssystem nach einem der Ansprüche 1 bis 3, wobei der expansionsfähige Einfangkorb (24) einen selbstexpandierenden metallischen Rahmen (54) und einen Maschenkörper (56) umfasst.

5. Selbstexpandierender Stent und Stentzuführungssystem nach Anspruch 4, wobei der expansionsfähige Einfangkorb (24) ein proximales und ein distales Ende aufweist, der selbstexpandierende Stent (20) ein proximales und ein distales Ende aufweist und der selbstexpandierende Stent (20) an dem länglichen Kernelement (14) proximal des expansionsfähigen Einfangkorbs (24) so angebracht ist, dass das proximale Ende des Einfangkorbs (24) von dem distalen Ende des Stents (20) beabstandet ist.

6. Selbstexpandierender Stent und Stentzuführungssystem nach Anspruch 5, wobei der Abstand zwischen dem proximalen Ende des expansionsfähigen Einfangkorbs (24) und dem distalen Ende des selbstexpandierenden Stents (20) im Bereich von etwa einem Millimeter bis zwei Zentimetern liegt.

7. Selbstexpandierender Stent und Stentzuführungssystem nach Anspruch 5, wobei der Abstand zwischen dem proximalen Ende des expansionsfähigen Einfangkorbs (24) und dem distalen Ende des selbstexpandierenden Stents (20) im Bereich von etwa fünf Millimetern bis fünfzehn Millimetern liegt.

8. Selbstexpandierender Stent und Stentzuführungssystem nach Anspruch 5, wobei das längliche Kernelement (14) die Form eines Drahtes annimmt.

9. Selbstexpandierender Stent und Stentzuführungssystem nach Anspruch 8, wobei das Anschlagelement (16, 18) die Form einer zylindrischen Spule annimmt, die um das längliche Kernelement (14) angeordnet ist.

## Revendications

1. Stent auto-expansible et système de mise en place de stent comprenant :
■ un cathéter à ballonnet (2) comprenant un cathéter allongé (3) ayant une lumière de mise en place (7), des sections proximale et distale (4, 10), et un ballonnet expansible (9) monté sur la section distale (10) dudit cathéter allongé (3) ;
■ un élément de noyau allongé (14) ayant des extrémités proximale et distale (22), ledit élément de noyau allongé (14) étant disposé de façon coulissante à l'intérieur de la lumière de mise en place (7) dudit cathéter allongé (3) ;
■ un stent auto-expansible (20) monté sur ledit élément de noyau allongé (14) ; et,
■ une cage de capture expansible (24) fixée à l'extrémité distale (22) dudit élément de noyau allongé (14), ladite cage de capture expansible (24) ayant pour but de piéger des débris emboliques qui peuvent se déloger d'un vaisseau sanguin affecté au cours d'une angioplastie du vaisseau sanguin.

2. Stent auto-expansible et système de mise en place selon la revendication 1, dans lequel ledit élément de noyau allongé (14) comprend un élément de butée (16, 18) s'étendant radialement vers l'extérieur dudit élément de noyau allongé (14), et ledit stent auto-expansible (20) se met en prise avec ledit élément de butée (16, 18), pour permettre ainsi audit stent auto-expansible (20) d'être déplacé de façon proximale et distale à travers ladite lumière de mise en place (7) dudit cathéter allongé (3) lorsque ledit élément de noyau allongé (14) est déplacé de façon proximale et distale à travers ladite lumière de mise en place (7) dudit cathéter allongé (3).

3. Stent auto-expansible et système de mise en place de stent selon la revendication 1 ou la revendication 2, dans lequel ladite cage de capture expansible (24) est une cage de capture auto-expansible (24).

4. Stent auto-expansible et système de mise en place de stent selon l'une quelconque des revendications 1 à 3, dans lequel ladite cage de capture expansible (24) comprend un cadre métallique auto-expansible (54) et un corps à mailles (56).

5. Stent auto-expansible et système de mise en place de stent selon la revendication 4, dans lequel ladite cage de capture expansible (24) comprend des extrémités proximale et distale, ledit stent auto-expansible (20) comprend des extrémités proximale et distale, et ledit stent auto-expansible (20) est monté sur ledit élément de noyau allongé (14), de façon proximale à ladite cage de capture expansible (24) de telle sorte que l'extrémité proximale de ladite cage de capture (24) soit espacée de l'extrémité distale dudit stent (20).

6. Stent auto-expansible et système de mise en place de stent selon la revendication 5, dans lequel la distance entre l'extrémité proximale de ladite cage de capture expansible (24) et l'extrémité distale dudit stent auto-expansible (20) est dans une plage d'environ un millimètre à deux centimètres.

7. Stent auto-expansible et système de mise en place de stent selon la revendication 5, dans lequel la distance entre l'extrémité proximale de ladite cage de capture expansible (24) et l'extrémité distale dudit stent auto-expansible (20) est dans une plage d'environ cinq millimètres à quinze millimètres.

8. Stent auto-expansible et système de mise en place de stent selon la revendication 5, dans lequel ledit élément de noyau allongé (14) prend la forme d'un fil.

9. Stent auto-expansible et système de mise en place de stent selon la revendication 8, dans lequel ledit élément de butée (16, 18) prend la forme d'une bobine cylindrique disposée au niveau dudit élément de noyau allongé (14).
